Europäisches Patentamt

European Patent Office

Office européen des brevets

(11) Publication number: **0 378 189 B1**

## EUROPEAN PATENT SPECIFICATION

(45) Date of publication of patent specification: **14.06.95**  (51) Int. Cl.6: **A61B  17/14**, B23D 61/02

(21) Application number: **90100422.6**

(22) Date of filing: **10.01.90**

(54) **Stepped surgical saw blade.**

(30) Priority: **13.01.89 US 296602**

(43) Date of publication of application:
**18.07.90 Bulletin  90/29**

(45) Publication of the grant of the patent:
**14.06.95 Bulletin  95/24**

(84) Designated Contracting States:
**BE DE ES FR GB IT NL SE**

(56) References cited:
**EP-A- 0 211 423**
**CH-A- 139 243**
**GB-A- 2 107 641**
**US-A- 4 036 236**
**US-A- 4 617 930**

**PATENT ABSTRACTS OF JAPAN, vol. 8, no.
158 (M-311)[1595], 21st July 1984;& JP-A-59
53 119 (FUJIKOSHI K.K.) 27-03-1984**

(73) Proprietor: **Pappas, Michael J.**
**61 Gould Place**
**Caldwell, N.J. 07006 (US)**

(72) Inventor: **Pappas, Michael J.**
**61 Gould Place**
**Caldwell, N.J. 07006 (US)**

(74) Representative: **LOUIS, PÖHLAU, LOHRENTZ &
SEGETH**
**Postfach 3055**
**D-90014 Nürnberg (DE)**

Rank Xerox (UK) Business Services
(3.10/3.09/3.3.3)

## Description

This invention relates to a surgical integral saw blade according to the preamble of claim 1.

Surgical saw blades for use with powered surgical saws are well known. Generally they are relatively thin (approximately 0,076 cm = 0,030 inches) surgical steel elements having staggered teeth on a cutting edge. The degree of stagger is often approximately half the material thickness, i.e. 0,038 cm (0,015 inches) above the plane of the top surface of the saw blade and 0,038 cm (0,015 inches) below the plane of the buttom surface of the surgical saw. Thus, because of the stagger, a saw which is 0,076 cm (0,030 inches) in thickness ordinarily makes a cut which is at least 0,152 cm (0,060 inches) thick.

Surgical saws so structured have presented a plurality of problems. When used with a flat guide surface they tend to flex in response to the efforts of the surgeon to hold the blade flat on the guide surface. The problem of flexing is particularly troublesome when the surgeon is performing "skimming" cuts, i.e. cuts where only very slight thicknesses of bone are to be removed. Thin surgical saw blades tend to flex and be deflected away from the desired plane of the cut. Such flexion results in surgical difficulties and worse, may result in uneven resectioned surfaces.

Another problem with the known blade structures is that they are not conducive for use with capture slot type guides, i.e. guides which are defined by slots rather than uni-planar surfaces. Where such slots are closed-ended, because of the saw tooth's stagger, the slot width must be substantially greater than the thickness of the blade so as to permit passage of the toothed portion therethrough.

Even where a capture slot is open at one end, the slot width must be substantially greater than the blade thickness or the guide containing the slot must be positioned away from the bone being resectioned to permit room for the staggered teeth of the blade to be introduced between the bone to be cut and the guide. Such displacement of the guide from the bone to be cut is recognized by most surgeons to be undesirable.

An apparently obvious solution to the flexion problem is to increase the blade thickness. In prior blade design philosophy, however, increasing the blade thickness was unacceptable because as the blade thickness increased, so did the tooth stagger tooth size and cut width. Thick staggered teeth are known to increase cutting effort and to generate excessive heat during cutting. Neither of these conditions is desirable.

A surgical integral saw blade according to the preamble of claim 1 is known from GB-A-2 107 641. According to this publication the stagggered teeth are formed, such as by stamping, into an uniformly thick blade so that the saw blade tends to flex, if it is very thin.

From US-A-4 617 930 it is known a saw blade having teeth in the same plane as the blade, i.e. the teeth are not staggered.

Further, this publication reveals a stiffener for the saw blade in such a way that the unsupported length of the stiffener is smaller than the length of the saw blade not supported by the stiffener.

It is an object of the present invention, therefore, to provide a surgical integral saw blade according to the preamble of claim 1 which is resistant to flexion during operation.

This object is solved by the features in the characterizing clause of claim 1.

Advantageous embodiments are stated in the sub-claims.

The claimed saw blade does not require unnecessary cutting force to be applied by the surgeon, does not generate unacceptable levels of heat during cutting, is suitable for use with a capture slot type guide and permits bone cuttings or other material to pass freely away from the saw.

## BRIEF DESCRIPTION OF THE DRAWING

A more complete understanding of the present invention may be had from the following detailed description thereof, particularly when read in view of the attached drawing, wherein:

FIGURE 1 is a plan view of a surgical saw blade structured according to the present invention;

FIGURE 2 is a side view of the saw blade of FIG. 1;

FIGURE 3 is an elevational view, partially in cross-section of a surgical saw blade utilized with a capture slot type surgical cutting guide to resection a bone; and

FIGURE 4 is an elevational view, partly in cross-section, of a prior art device.

## DETAILED DESCRIPTION

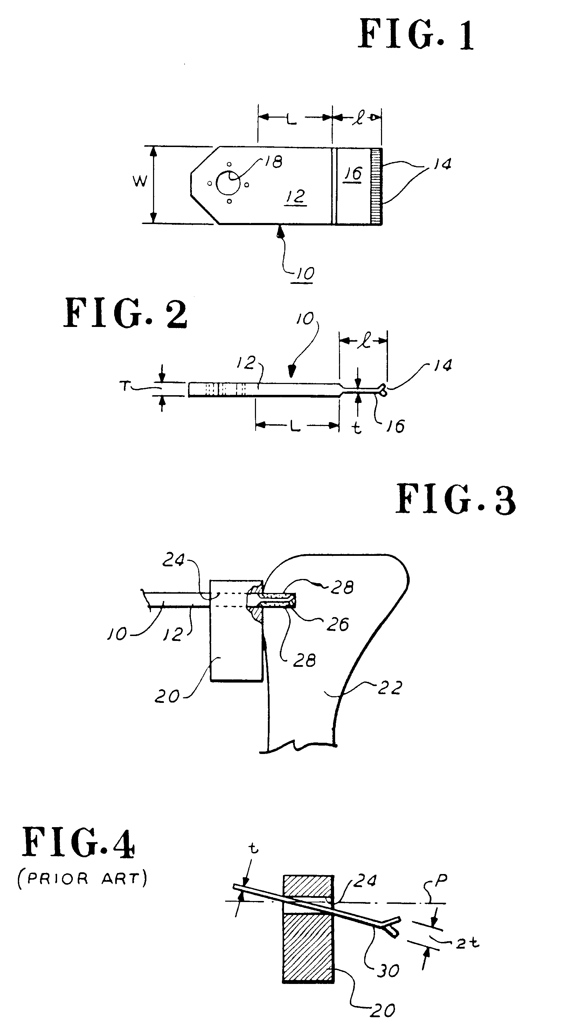

Referring therefore to FIGURES 1 and 2, a surgical saw structured in accordance with the teaching of the present invention is shown and designated generally by the reference numeral 10.

Saw 10 can be seen to include a base blade portion 12, a plurality of staggered teeth 14 and an intermediate blade portion 16. Provided at the end of saw 10 which is remote from staggered teeth 14 are a plurality of openings 18 which will be recognized by those skilled in these arts to define a securing means whereby saw blade 10 may be locked to a power surgical saw (not shown).

The width of saw 10 is uniform and designated here by letter "w." The area of the base blade portion 12 of saw 10 unsupported by the saw is disclosed to be of a length "L " with a thickness designated by the letter "T". A typical thickness for such a saw blade would be where the dimension "T" is approximately 0,152 cm (0,060 inches). In this regard, it has been found that such a thickness is sufficient to avoid most of the flexion problems discussed above with respect to prior art blades.

The intermediate blade portion 16 is shown to be of a length "l " and a thickness designated by the letter "t". It has been found that a saw having a base blade thickness of 0,152 cm (0,060 inches) is well served by an intermediate blade portion 18 which is 0,076 cm (0,030 inches) thick. In this regard where intermediate blade portion 16 is 0,076 cm (0,030 inches) thick, the dimension from the top edges of staggered teeth 14 to the bottom edges of staggered teeth 14 may be equal to or slightly greater then double the thickness "t", i.e. slightly greater than the thickness "T". It has also been found that it is desirable that the length "l" be no greater than the length "L " to avoid undesirable flexions although preferably length "l" should be only long enough to allow the passage of bone cuttings.

Reduction of the thickness of saw blade 10 so as to define intermediate blade portion 16 provides two benefits. First, it permits the formation of staggered teeth 14, such as by stamping so as to result in a tooth depth, i.e., the distance from the top edges of the upwardly extending staggered teeth 14 to the bottom edges of downwardly extending staggered teeth 14, which may be equal to or slightly greater than the thickness "T" of base blade portion 12. Where larger or smaller tooth depths are desired, the forming technique can be adjusted accordingly. Secondly, the reduced thickness of the intermediate blade portion 16 provides a space into which cutting chips may be released from the saw teeth. This is particularly beneficial where the cutting blade extends into the member being cut. In such instances, the cutting chips are displaced away from the teeth and pass transversely of the blade out of the resected area. The clearing of the chips, of course, is assisted by the motion of the blade.

The advantage of surgical saw blades structured in accordance with the present invention is that the thickness of the base blade portion 12 provides resistance to deflection while the reduced thickness of the intermediate blade portion 16 permits forming of the staggered teeth at any desired thickness, including a thickness equal to or less than the thickness of the base blade portion 12. Further, the reduced thickness of the intermediate blade portion 16 facilitates removal of cutting chips, as discussed above.

Saw blade 10 may be manufactured using any of the many materials known to those skilled in these arts. Further, saw blade 10 may be manufactured using any of the manufacturing techniques known to those skilled in these arts.

The true advantages of saw blade 10 best may be seen with reference to FIGS. 3 and 4. More specifically, FIG. 3 shows a saw blade 10 in use with a cutting guide 20 to cut a bone such as a tibia 22. Cutting guide 20 is provided with a capture slot 24 through which blade 10 is inserted. FIG. 4 shows a prior art saw 30 in capture slot 24. Both saws 10 and 30 have identical tooth portion thickness "2t." With the conventional saw 30 since the slot 24 is much wider than the thickness "t," the saw 30 is free to move out of the guide plane "P" and thus can produce a resection plane substantially different than desired.

As best may be seen from FIG. 3, the height "H" of capture slot 24 is only very slightly greater than the thickness "T" of the base blade portion 12 of saw 10. Thus, blade 10 is supported against flexion by both the upper and lower surfaces of the capture slot 24. Also, the depth "D" of the capture slot 24 is substantial. Additionally, the length "l of the intermediate portion 16 should be substantially less than the depth of the capture slot 24 so that there will be sufficient support for the base blade portion 12 by slot 24.

As blade 10 forms a cut 26 in the bone 22, chips or bone fragments 28 resulting from the cutting are carried away from the cutting face and are carried out of the cutting area by the oscillation of the cutting blade 10. As a result the cutting face is maintained clean and the cutting process can continue without reservation as to interference from build-up of chips 28.

The stiffness of the saw 10 may be adequately computed using the simple cantilever beam bending equation which for this case is:

$$K = E \, w \, T_e^3 / 4(L + l)^3$$

where

| | |
|---|---|
| K = | stiffness of the saw 10 |
| $T_e$ = | effective thickness of saw 10 where "$T_e$" is less than "T" and greater than "t" and where "$T_e$" is a function of "L " and "l" |
| w = | width of saw 10 |
| (L + l) = | unsupported length of saw 10 |
| E = | the material stiffness (Young's modulus) |

From this equation it may be seen that given a particular saw length (L + l) and width "w" that a saw 10 having an effective thickness, "$T_e$" equal to 0,152 cm (0,060 inches) is eight times stiffer than a saw 0.076 cm (0,030 inches) thick which is typical for surgical saws. Since the stiffness "K" is proportional to the unsupported length cubed, $(L + l)^3$, when the length "l" of portion 16 is made small, but sufficient to clear bone cutting chips 28, the effective thickness "$T_e$" will approach "T" and the portion 16 will have little effect on overall stiffness "K." Thus a saw 10 of the type taught here can be made approximately eight times stiffer than a conventional saw while still retaining the cutting characteristics of the conventional saw with respect to cut width, forces and heat generation.

Conventional saws have been observed to bend more than 0,203 cm (0,080 inches) during resection producing cuts which thus differed from the desired resection plane by that amount. This level of error is undesirable and often unacceptable. An acceptable error for many applications, such as preparation of flat surfaces to receive prostheses loading surfaces, is about 0,051 cm (0,020 inches) or one quarter the observed amount. Thus a saw stiffness "K" of about four times the stiffness of a typical conventional saw is highly desirable for many applications. Such stiffness "K" can be achieved with the type of saw taught here even where the length "l" of the intermediate portion 16 is approximately the same length as the base portion 12. Thus from a stiffness perspective one can produce a saw 10 of satisfactory stiffness "K" where the length "L " of the base portion 12 is approximately equal to the length "l" of the intermediate portion 16.

Where the saw 10 is used in conjunction with the guide 20, however, it is desirable to use a relatively short, thin portion 16 so that the thicker portion 12 can act as a uniform guiding surface during the resection procedure. Thus the length " l" of the thin portion 16 should generally be much less than the width of the guide 20 with which it is used.

There are two further advantages to the saw 10 taught here. First, as a result of the increased stiffness of the saw 10 it is possible now to produce a saw 10 of adequate stiffness which makes a thinner cut and uses finer teeth 14 (the tooth size is dependent on the saw thickness "t" increasing with increasing thickness), thereby producing a saw 10 which requires less force to produce a resection and which generates less heat during resection. For example, if one can produce a saw 10 that is eight times stiffer than a conventional saw, and if a saw of only four times greater stiffness is needed for a given application, one can then make a saw for this application according to this teaching which has the desired stiffness "K" but which will make a thinner cut than a conventional saw with its attendant benefits. Further, in those cases where a conventional saw is of adequate stiffness then it is possible, using these teachings, to produce a saw which will make a cut only half the thickness of a cut made by a conventional saw thereby greatly reducing needed cutting forces and bone damaging heat.

Second, the thick portion 12 helps guide the saw 10 even in the absence of a guide 20. Frequently in cutting bones, the surgeon first uses a guide 20 to help make a partial cut. The guide 20 is then removed and the partial cut is used as a "miter box" to guide the saw 10 to complete the cut. In this situation, the partial cut acts like a capture slot in a guide instrument and thus for the same reasons that the saw 10 taught here provides greater cutting precision when used with the capture slot 24, it provides greater cutting precision when used with the partial cut "miter box." Further, a stiffer saw 10 requiring less cutting force than a conventional saw is easier to direct and control than a conventional saw.

It is clear, therefore, that the surgical saw blade 10 of the present invention provides a structure which is resistant to undesirable flexion and which also may be utilized with capture slots without the attendant problems set out above.

**Claims**

1.  A surgical integral saw blade comprising:
    a base blade portion (12) having a thickness (T), said base blade portion including a supported length which is adapted to be engaged by and supported by an operating means such as a saw head, and an unsupported length (L);
    a plurality of staggered teeth (14) displaced from said base blade portion; and an intermediate blade portion (16),
    characterized in that the thickness (t) of said intermediate blade portion (16) being less than the thickness (T) of said base blade portion (12), wherein the distance from the top edges of the upwardly extending staggered teeth (14) to the bottom edges of the downwardly extending staggered teeth is substantially equal to the thickness (T) of said base blade portion (12) and wherein said staggered teeth (14) are displaced from said base blade portion (12) by an amount (l) no more than the unsupported length (L) of said base blade portion (12)

2.  A surgical saw according to claim 1 wherein the thickness (t) of said intermediate blade portion (16) is sufficiently less than the thickness (T) of said base blade portion (12) to permit passage of cuttings away from said staggered teeth (14) during operation of the said surgical saw when said staggered teeth (14) are disposed within a member being cut.

**3.** A surgical saw according to one of the claim 1 or 2 further including a guide (20) having a capture slot (24) therein and wherein said base blade portion (12) slideably extends into said slot (24) with the length (l) of said intermediate blade portion (16) being less than the depth (D) of said slot.

## Patentansprüche

**1.** Einstückig ausgebildetes chirurgisches Sägeblatt umfassend:

Einen Basisblattabschnitt (12) mit einer Dicke (T), wobei der Basisblattabschnitt einen abgestützten Längsabschnitt aufweist, der so beschaffen ist, daß er von einem Betätigungsmittel, wie einem Sägenkopf, gegriffen und gehalten werden kann, und einen nicht abgestützten Längsabschnitt (L);

eine Vielzahl von zueinander versetzten Zähnen (14), die von dem Basisblattabschnitt beabstandet sind; und einen Zwischenblattabschnitt (16),

**dadurch gekennzeichnet,**

daß die Dicke (t) des Zwischenblattabschnitts (16) geringer ist als die Dicke (T) des Basisblattabschnitts (12), wobei der Abstand von den oberen Kanten der nach oben erstreckten versetzten Zähne (14) zu den unteren Kanten der nach unten erstreckten versetzten Zähne im wesentlichen der Dicke (T) des Basisblattabschnitts (12) entspricht, und wobei die zueinander versetzten Zähne (14) von dem Basisblattabschnitt (12) einen Abstand (l) aufweisen, der nicht größer ist als der nicht abgestützte Längsabschnitt (L) des Basisblattabschnitts (12).

**2.** Chirurgische Säge nach Anspruch 1,

**dadurch gekennzeichnet,**

daß die Dicke (t) des Zwischenblattabschnitts (16) hinreichend geringer ist als die Dicke (T) des Basisblattabschnitts (12), um den Austrag von durch die zueinander versetzten Zähne (14) abgetragenen Schnittguts während der Benutzung der chirurgischen Säge zu erlauben, wenn sich die zueinander versetzten Zähne (14) innerhalb eines zu schneidenden Teils befinden.

**3.** Chirurgische Säge nach Anspruch 1 oder 2, gekennzeichnet durch eine Führung (20) mit einem darin ausgebildeten Aufnahmeschlitz (24), wobei sich der Basisblattabschnitt (12) gleitend in den Schlitz (24) hineinerstreckt und wobei die Längserstreckung (l) des Zwischenblattabschnitts (16) geringer ist als die Tiefe (D) des Schlitzes.

## Revendications

**1.** Lame pour scie chirurgicale en une pièce, comprenant :
- une portion de base (12) ayant une épaisseur (T)
- cette base présentant une longueur portée en contact avec et portée par des moyens d'entraînement tels qu'une tête de scie, et une longueur non portée (2),
- un certain nombre de dents avoyées (14) décalées par rapport à cette base, et
- une portion de lame intermédiaire (16),

caractérisée en ce que l'épaisseur (t) de ladite portion de lame intermédiaire (16) est inférieure à l'épaisseur (T) de la portion de base (12), la distance des bords supérieurs des dents avoyées vers le haut (14) aux bords inférieurs des dents avoyées vers le bas étant pratiquement égale à l'épaisseur (T) de la portion de base (12), et les dents avoyées (14) étant éloignées par rapport à ladite portion de base (12) d'une distance (l) qui n'est pas supérieure à la longueur non portée (L) de ladite portion de base (12).

**2.** Scie chirurgicale selon la revendication 1, dans laquelle l'épaisseur (t) de ladite portion de lame intermédiaire (16) est suffisamment inférieure à l'épaisseur (T) de ladite portion de base (12) pour permettre le passage des copeaux expulsés par les dents avoyées (14) au cours de l'utilisation de ladite scie chirurgicale, quand lesdites dents avoyées (14) sont situées à l'intérieur d'un membre soumis à une découpe.

**3.** Scie chirurgicale selon l'une des revendications 1 ou 2, comportant au surplus un guide (20) présentant une fente de maintien (24), la portion de base de lame (12) coulissant à l'intérieur de ladite fente, la longueur (l) de ladite portion de lame intermédiaire (16) étant inférieure à la profondeur (D) de ladite fente.

# FIG. 1

# FIG. 2

# FIG. 3

# FIG. 4
(PRIOR ART)